# EUROPEAN PATENT APPLICATION

(11) **EP 0 845 246 A1**
(43) Date of publication of application: **03.06.1998**
(21) Application number: 97203678.4
(22) Date of filing: 27.11.1997
(51) Int. Cl.: A61B 19/00

(54) **Surgical drape**

(30) Priority: 28.11.1996 FI 960590 U
(71) Applicant: Uudenmaan Sairaalapesula OY, 04250 Kerava (FI)
(72) Inventor: Lintukorpi, Anne, 4230 Kerava (FI)
(74) Representative: Heinänen, Pekka Antero

(57) **Abstract**

The invention relates to a surgical drape for covering a patient at least partially prior to a surgical operation, said surgical drape being formed from at least two pieces (1-4) mutually attached to each other. The invention is implemented so that, in order to make the drape to fall in an improved manner at a desired point, e.g., at the sideways extended arms, a first portion (1) of the drape is provided by cutting or otherwise shaping to have a rectangular inward angled edge (5, 6), and that to said rectangular inward angled edge, advantageously by sewing, is attached the straight edge of a second portion (3) of the surgical drape.

## Description

The present invention relates to a surgical drape for covering a patient at least partially prior to a surgical operation, said surgical drape being comprised of at least two parts attached to each other.

The covering of a patient before undertaking a surgical procedure is technique aiming to restrict the access of harmful agents into the body of the patient during the procedure, generally involving a cut or puncture made to the skin and/or mucous parts of the patient. Desirably, the extended set of drapes forms a barrier layer between the actual area of surgical operation enclosing the skin cut/puncture and other parts of the patient's skin or nonsterile materials of the surrounding furniture and other similar equipment.

The provision of a contiguous protective layer requires that the set of drapes may either comprise a contiguous drape as such, or alternatively, will be integrally formed into an entity from drape portions tightly seamable to each other. Herein, a problem arises particularly from the rectangular area spanned by the patient's torso and the laterally extended arms. Here, drapes of a rectangular shape do not fall coveringly into the angle subtended between the torso and the stretched arm, whereby the arm support and/or the operating room table remain visible. This situation forms an aseptic risk and a possibility of contamination involving the access of harmful agents into the surgical wound.

It is an object of the present invention to provide a novel type of surgical drape free from the above-described problems. The surgical drape according to the invention is characterized in that, in order to make the drape to fall in an improved manner at a desired point, e.g., at the sideways extended arms, a first portion of the drape is provided by cutting or otherwise shaping to have a rectangular inward angled edge, and that to said rectangular inward angled edge, advantageously by sewing, is attached the straight edge of a second portion of the surgical drape. When the surgical drape according to the invention is unfolded in place, the "excess" drape remains slack particularly about the rectangular inward angled edge, whereby the surgical drape falls coveringly also at the difficult-to-cover areas situated about the limbs.

An embodiment of the invention is characterized in that, prior to the sewing of said second portion of the surgical drape, the edge of said first portion of the surgical drape, to which the rectangular inward angled edge is formed, is extended straight for sewing the second portion thereto.

In the following, the invention will be examined in greater detail by making reference to the appended drawing in which is shown a surgical drape put together from a number of textile pieces, particularly illustrating the so-called upper surgical drape used for covering the patient's upper torso.

As shown in the diagram, the surgical drape according to the invention is comprised of a number of textile pieces 1-4 sewn or otherwise attached to each other. The textile material may be selected freely from the grades conventionally used in the art. Accordingly, the drape textile can be, e.g., a two- or multilayer laminated material, typically a three-layer laminated fabric, having laminated between the two outer material layers a membrane which is impervious to liquids, germs and vira, but is pervious to moisture. The use of such a material is particularly important in areas close to the area of the surgical operation. For those areas of the drape which are farther removed from the surgical operation area, the surgical drape can be made from some other textile such as a tightly knit polyester fabric or a nonwoven two-layer laminate. In a multipurpose drape, the permeability of the drape material to moisture facilitates, on one hand, sterilization of the drape material and, on the other hand, balanced control of patient body temperature during the operation. Alternatively, the surgical drape can be made from some other type of material, such as a nonwoven fabric and a plastic laminate.

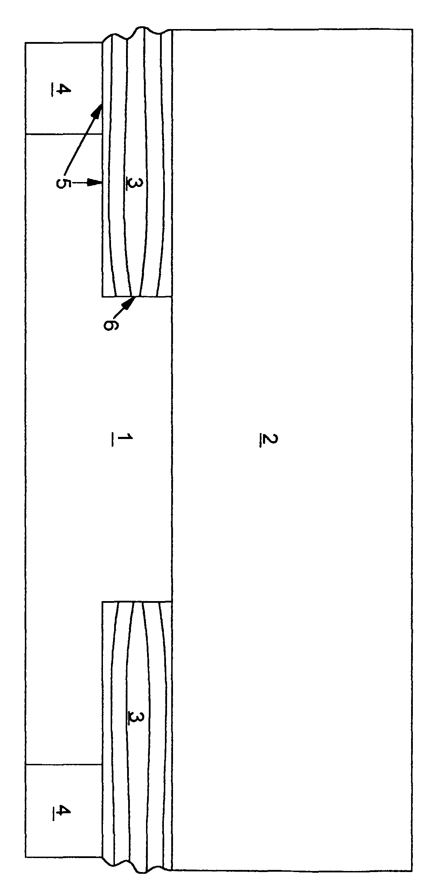

In the embodiment illustrated in the drawing, the surgical drape, particularly the so-called upper drape described herein comprises a center piece 1 having rectangular, possibly square-shaped, pieces cut off from both of its upper corners. The center piece 1 is extended at both of its side edges with sewn lower side pieces 4. This particular layout is advantageous in terms of series production. Without departing from the scope of the invention, the center piece 1 and the lower side pieces 4 may also comprise a single, contiguous piece of fabric. As shown in the diagram, above the center piece 1 is sewn an upper piece 2. Between the upper piece 2 and the center piece 1 are sewn intermediate side pieces 3. In the unfolded position of the surgical drape shown in Fig. 1, the intermediate side pieces 3 remain "slack", thus permitting them to fall freely downward about the hands of the patient so as to cover the desired areas such as the arm support and the corresponding area of the operating room table.

The "slackness" of the intermediate side pieces is achieved by sewing a rectangular piece of fabric to the edges 5, 6 of the center piece 1 and the lower side piece 4 so that said edges are aligned so as to form a continuous line during sewing. In practice, the edge 5 is stretched into straight continuation of the edge 6. Successively, one straight edge of the intermediate side piece 3, advantageously having a length equal to the overall length of the stretched edge 5, 6, is sewn to said stretched edge 5, 6. When the edge 5 after sewing is folded back into its initial position, which is orthogonal to the edge 6 in the operating position of the drape as shown in the drawing, the intermediate side piece 3 remains "slack", whereby its overall area becomes appreciably larger than the projected geometrical area of the intermediate side piece in the drawing (computed as length of edge 5 times length of edge 6). The right-hand intermediate side piece is sewn in place using exactly the same technique. Conventional sewing techniques are used for sewing the upper edges of the intermediate side pieces 3 to the edge of the upper piece 2.

To those versed in the art, it is obvious that the invention is not limited by the exemplifying embodiment described above, but rather can be varied within the scope and spirit of the appended claims. For instance, the acute angle between the edges 5 and 6 is advantageously made to be 90° as accurately as possible in the operating position of the surgical drape. While small deviations from this angle could be contemplated in theory, other kinds of layouts are not practicable in series production. Obviously, a surgical drape may be assembled from pieces of other shapes and sizes than those described in the exemplifying embodiment. However, according to an essential specification of the invention, the drape portion called the center piece is provided with an essentially rectangularly inward angled edge, which is stretched into a straight edge prior to sewing the intermediate side piece thereto, whereby the straight edge of said intermediate side piece can be directly sewn to the "straight-folded" edge 5, 6 of said center piece. Naturally, the surgical drape discussed herein need not be the above-mentioned upper drape, but rather, the drape may be any type of surgical garment needing such "slack" areas in its layout for improved falling of the fabric. Hence, the above-used terms "center piece", "lower side piece", "intermediate side piece", etc., must be understood being related to the elucidation of the function of the exemplifying embodiment only. Therefore, the use of the terms is nonlimiting to the placement of the fabric pieces in the contemplation of other possible embodiments of the invention.

## Claims

1. A surgical drape for covering a patient at least partially prior to a surgical operation, said surgical drape being formed from at least two pieces (1-4) mutually attached to each other, **characterized** in that, in order to make the drape to fall in an improved manner at a desired point, e.g., at the sideways extended arms, a first portion (1) of the drape is provided by cutting or otherwise shaping to have a rectangular inward angled edge (5, 6), and that to said rectangular inward angled edge, advantageously by sewing, is attached the straight edge of a second portion (3) of the surgical drape.

2. A surgical drape as defined in claim 1, **characterized** in that, prior to the sewing of said second portion (3) of the surgical drape, the edge (5, 6) of said first portion (1) of the surgical drape, to which the rectangularly inward angled edge is cut, is extended straight for sewing said second portion (3) of the surgical drape thereto.
